# EUROPÄISCHE PATENTSCHRIFT

(11) **EP 0 401 515 B1**
(45) Veröffentlichungstag und Bekanntmachung des Hinweises auf die Patenterteilung: **02.08.1995**
(21) Anmeldenummer: 90108495.4
(22) Anmeldetag: 07.05.1990
(51) Int. Cl.: A61M 5/142, A61K 9/22

(54) **Implantierbare Vorrichtung zur dosierten Abgabe von Medikamenten in den menschlichen Körper**
Implantable device for dispensing measured amounts of medicaments in the human body
Dispositif implantable pour l'administration dosée de médicaments dans le corps humain

(30) Priorität: 10.05.1989 DE 3915251
(43) Veröffentlichungstag der Anmeldung: 12.12.1990
(73) Patentinhaber: Fresenius AG, 61350 Bad Homburg v.d.H (DE)
(72) Erfinder:
(74) Vertreter: Fuchs Mehler Weiss

(56) Entgegenhaltungen:
- EP-A- 0 083 319
- EP-A- 0 134 758
- EP-A- 0 347 743
- WO-A-84/01718
- WO-A-87/04631
- DE-A- 3 806 008
- US-A- 4 820 273

## Beschreibung

Die Erfindung betrifft eine implantierbare Vorrichtung zur dosierten Abgabe von Medikamenten in den menschlichen Körper gemäß Oberbegriff des Anspruchs 1.

Eine implantierbare Vorrichtung ist aus der AT-PS 1631/88 bekannt. Diese Vorrichtung weist ein Gehäuse auf, in dem eine erste Speicherkammer für das zu verabreichende Element angeordnet ist, die über ein durchstechbares Septum nachgefüllt werden kann und die über eine Auslaßöffnung und eine Auslaßreduziereinrichtung in Form einer Schlauchwindung mit einer ringförmigen Kammer verbunden ist, die zu einem Auslaßkatheter führt. Das in der ersten Kammer befindliche Medikament wird durch Druckaufbringung dem Auslaßkatheter zugeführt, wozu eine zweite Kammer vorgesehen ist, die mit einem sich bei Körpertemperatur ausdehnenden Treibmittel gefüllt ist und die durch eine Membran von der ersten Kammer getrennt ist.

Derartige implantierbare Vorrichtungen bzw. Pumpen werden zur kontinuierlichen Medikation bei Patienten eingesetzt, die ansonsten nur durch Spritzen von Medikamenten mehrmals täglich behandelt werden könnten. Hierbei müssen insbesondere bei der Gabe von Schmerzmittel an derartige Pumpen hohe Sicherheitsanforderungen gestellt werden, da derartige Schmerzmittel häufig bei Überdosierung tödlich wirken und darüber hinaus auch ein Mißbrauch verhindert werden muß.

Bei der gattungsgemäßen Vorrichtung wie auch bei anderen ähnlich aufgebauten Pumpen tritt das Problem auf, daß es bei der Einschleppung von Keimen zu einer starken Keimvermehrung kommen kann und daß Partikel, die beim Durchstechen des Septums entstehen, entweder die Auslaßreduziereinrichtung oder den Auslaßkatheter verstopfen können.

Um dies zu vermeiden, ist es zwar bei einigen bekannten Pumpen vorgeschlagen worden, entweder zwischen einer Eingangskammer oder dem Reservoir oder zwischen dem Reservoir und dem Pumpmechanismus Filter einzubauen oder auch die Leitung, die vom Reservoir zum Pumpenmechanismus führt, reservoirseitig mit einer Reihe von Löchern zu versehen. Im letzteren Falle sind jedoch diese Löcher oder Poren so groß, daß sie Bakterien nicht zurückzuhalten vermögen, und überdies sind die genannten Poren bei dieser Anordnung lediglich dazu vorgesehen, eine Blockierung der Ansaugöffnung bei kollabierendem Vorratsgefäß zu vermeiden. Die zuvorgenannten Pumpen (siehe z.B. WO 87/04631, von welcher der Oberbegriff des Anspruchs 1 ausgeht) verwenden Flachmembranfilter, die dicht in einer Haltevorrichtung eingeklemmt sind. Diese Konstruktion erfordert jedoch viel Platz, der bei einer implantierbaren Pumpe jedoch natürlich möglichst klein gehalten werden muß. Darüber hinaus ist das Abdichten der Membran und die Prüfung der Dichtheit schwierig. Daher ist das eingangs genannte Problem bisher nicht zufriedenstellend gelöst worden.

Es ist daher Aufgabe der vorliegenden Erfindung, eine implantierbare Vorrichtung der im Oberbegriff des Anspruchs 1 angegebenen Art zu schaffen, bei der es möglich ist, die Gefahr des Verstopfen der Auslaßreduziereinrichtung oder des Auslaßkatheters zu vermeiden.

Die Lösung dieser Aufgabe erfolgt durch die Merkmale des Anspruches 1.

Durch das Vorsehen eines Hohlfaserkapillarfilters, der an einem Ende z. B. mit der Auslaßreduziereinrichtung verbunden ist, wird es möglich, in die erste Kammer eingedrungene Partikel sicher am Eindringen in die Auslaßreduziereinrichtung und den Auslaßkatheter zu hindern, so daß Verstopfungen dieser beiden Teile vorgebeugt wird. Dies erhöht erheblich die Funktionssicherheit der erfindungsgemäßen Vorrichtung.

Die implantierbare Vorrichtung gemäß vorliegender Erfindung weist ferner den Vorteil auf, daß die beschriebene Abdichtung auf einfache Art und Weise hergestellt werden kann, ohne daß ein zusätzliches Abdichtungsproblem entsteht. Darüber hinaus benötigt die Hohlfaserkapillare wenig Platz und läßt sich in einer oder mehreren Windungen in der ersten, den Vorratsbehälter bildenden Kammer anordnen, wodurch das Risiko der Verstopfung unter der Einwirkung von sich unter Gravitationswirkung bevorzugt an einer Stelle der ersten Kammer ablagernden Partikel vermieden wird.

Die Unteransprüche haben vorteilhafte Weiterbildungen der Erfindung zum Inhalt.

Weist die implantierbare Vorrichtung eine Durchlaßöffnung auf, über die die erste Kammer mit der Auslaßreduziereinrichtung verbunden ist, wird die zuvor mit der Auslaßreduziereinrichtung verbundene Hohlfaserkapillare durch die Durchlaßöffnung in die erste Kammer geführt, in der sie dann mit einer oder mehreren Windungen angeordnet wird.

Bei einer besonders bevorzugten Ausführungsform wird die Hohlfaserkapillare im Innern der ersten Kammer in eine Nut eingelegt, die oberhalb der Membran angeordnet ist. Dadurch wird verhindert, daß die Hohlfaserkapillare bei abnehmendem Medikamentenvorrat durch die Membran verschlossen wird, was noch durch die Ausgestaltung der Membran und des Innenraums der ersten Kammer unterstützt werden kann.

Durch das Bilden einer oder mehrerer Windungen kann die Filtrationsfläche der mikroporösen Hohlfaserkapillare erheblich erhöht werden, so daß sicher vermieden werden kann, daß die Hohlfaserkapillare selbst durch die in der ersten Kammer befindlichen Partikel verstopft wird.

Bevorzugterweise weist die Hohlfaserkapillare einen Innenduchmesser von etwa 200 »m und einen Außendurchmesser von etwa 280 »m auf. Ist die Auslaßreduziereinrichtung als Reduzierkapillare ausgebildet, kann entweder ein Ende dieser Reduzierkapillare über das entsprechende Verbindungsende der Hohlfaserkapillare geschoben werden, oder es wird in umgekehrter Weise das entsprechende Ende der Hohlfaserkapillare einige mm über das Ende der Reduzierkapillare geschoben, wonach die beiden Kapillarenden mit Hilfe eines Klebers, vorzugsweise Polyurethan, verbunden werden.

Bevorzugterweise kann als Hohlfaserkapillarfilter entweder eine übliche Dialysatormembran oder aber eine zur Plasmafiltration oder Plasmafraktionierung verwendete Membran höherer Ausschlußgrenze verwendet werden. Eine derartige Membran ist üblicherweise an einem ihrer stirnseitigen Enden verschlossen.

Weitere Einzelheiten, Merkmale und Vorteile der Erfindung ergeben sich aus nachfolgender Beschreibung eines Ausführungsbeispiels anhand der Zeichnung.

Es zeigen:
- Fig. 1: einen Vertikalschnitt durch eine erfindungsgemäße Vorrichtung und
- Fig. 2: einen Schnitt entlang der Linie I-I gemäß Fig. 1.

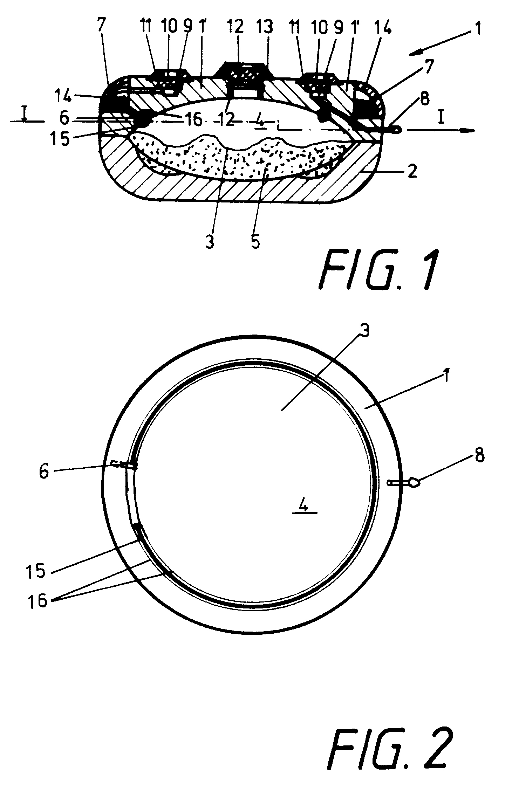

In Fig. 1 ist eine erfindungsgemäße implantiere Vorrichtung bzw. Pumpe 1 zur dosierten Abgabe von Medikamenten in den menschlichen Körper dargestellt. Die Vorrichtung 1 weist ein Gehäuse auf, das aus zwei Gehäuseteilen 1′ und 2 zusammengesetzt ist und dessen Innenraum durch eine flexible Membran 3 in eine erste Kammer 4 und eine zweite Kammer 5 geteilt ist. Die erste Kammer 4 dient hierbei zur Aufnahme des abzugebenden Medikamentes, während die zweite Kammer 5 dazu dient, über die Membran 3 einen Druck auf das in der ersten Kammer 4 befindliche Medikament auszuüben. Hierzu kann die zweite Kammer 5 beispielsweise mit einem Treibmittel gefüllt sein, welches sich durch die Körperwärme isobar ausdehnt. Dabei wird durch das Ausdehnen des Treibmittels die Membran 3 mit Druck beaufschlagt, so daß sich das Volumen der ersten Kammer 4 verkleinert und hierdurch das Medikament aus der Kammer 4 verdrängt wird. Dabei wird das Medikament über eine Auslaßöffnung 6, die mit der Kammer 4 in Verbindung steht, eine mit der Auslaßöffnung 6 in Verbindung stehende Auslaßreduziereinrichtung 7 und einen Auslaßkatheter 8 in den Körper des Patienten abgegeben. Bevor das Medikament den Auslaßkatheter 8 erreicht, wird es in eine Kammer 9 eingeleitet, welche ringförmig im Teil 1' des Gehäuses angeordnet ist. Bei der dargestellten Ausführungsform ist diese Kammer 9 an ihrer Oberseite durch einen Ring 10, welcher von einer Injektionsnadel durchstochen werden kann und der sich nach Herausziehen der Injektionsnadel selbsttätig wieder abdichtet, abgeschlossen. Der Ring 10 wird über eine ringförmige Befestigungseinrichtung 11 in seiner Lage gehalten.

Die erste Kammer 4 weist ferner eine Nachfüllöffnung 12' auf, die von einem zweiten Septum 12 geschlossen ist, welches mittels eines Befestigungsteiles 13 festgehalten ist. Dieses Septum 12 kann ebenfalls von einer Injektionsnadel zum Nachfüllen von Medikamenten in die erste Kammer 4 durchstochen werden.

Bei der in Fig. 1 dargestellten Ausführungsform ist die implantierbare Vorrichtung 1 als Rotationskörper ausgebildet, wobei die Nachfüllöffnung 12′ zentrisch angeordnet ist und die ringförmige Kammer 9 konzentrisch um die Nachfüllöffnung 12′ angeordnet ist.

Die Auslaßreduziereinrichtung 7 ist bei der in den Fig. 1 und 2 dargestellten Ausführungsform als Schlauchwindung oder als Reduzierkapillare ausgebildet, welche mehrfach in einer Ausnehmung des Teiles 1′ des Gehäuses herumgewunden ist. Die Ausnehmung ist durch einen Deckel 14 so abgedichtet, daß eine glatte äußere Kontur der implantierbaren Vorrichtung 1 erzielt ist.

Die Kammer 9 und damit der Abdichtring 10 ist konzentrisch um die Nachfüllöffnung 12′ herum angeordnet.

Im implantierten Zustand gibt die Vorrichtung bzw. Pumpe 1 über den Auslaßkatheter 8 kontinuierlich das in der ersten Kammer 4 befindliche Medikament an den Körper des Patienten ab. Soll aus gewissen gesundheitlichen Gründen mehr von diesem Medikament oder ein anderes zusätzliches Medikament an den Körper des Patienten abgegeben werden, wird mittels einer Injektionsspritze, deren Einstichort von einer in den Fig. nicht näher dargestellten, auf den Körper aufgesetzten Schablone bestimmt wird, der Ring 10 durchstochen und das abzugebende Medikament in die Kammer 9 injiziert, von welcher es unmittelbar über den Auslaßkatheter 8 an den Körper abgegeben wird. Der Flußwiderstand in der Auslaßreduziereinrichtung 7 ist dabei so groß, daß ein Zurückdrängen des Medikamentes in die erste Kammer 4 der implantierbaren Vorrichtung verhindert ist.

Wie eingangs beschrieben, kann es jedoch beim Durchstechen des Septums 12 oder des Septums 10 zu einem Partikelanfall kommen, der entweder die Auslaßreduziereinrichtung 7 oder den Auslaßkatheter 8 oder beide Teile verstopfen kann, was die Funktionsfähigkeit einer derartigen Vorrichtung herabsetzen bzw. zunichte machen könnte. Aus diesem Grund weist die erfindungsgemäße Vorrichtung 1 einen zusätzlichen Filter in Form einer Hohlfaserkapillare 15 auf, der an einem Ende mit dem Eintrittsende der Auslaßreduziereinrichtung verbunden ist und bei der in Fig. 1 dargestellten Ausführungsform durch die Auslaßöffnung 6 in das Innere der ersten Kammer 4 eingeleitet ist. In der Kammer 4 ist eine Nut 16 oberhalb der Membrane 3 angeordnet, in den der in die erste Kammer 4 eingeführte Abschnitt der Hohlfaserkapillare 15 eingelegt werden kann.

Wie Fig. 1 verdeutlicht, ist die Nut 16 oberhalb der Membrane 3 angeordnet, so daß ein Verschließen des in der Kammer 4 befindlichen Bereiches der Hohlfaserkapillare bei abnehmendem Medikamentenvorrat verhindert werden kann.

Fig. 2 verdeutlicht hierbei, daß bei dieser Ausführungsform eine Windung des Bereiches der Hohlfaserkapillare 15, der in die Kammer 4 eingeführt wurde, gebildet wird. Es ist jedoch grundsätzlich auch möglich, daß mehrere derartige Windungen zur Erhöhung der Filterfläche ausgebildet werden.

Wie beschrieben, ist bei der in den Fig. 1 und 2 beispielhaft verdeutlichten Vorrichtung 1 eine Auslaßreduziereinrichtung vorgesehen, mit der ein Ende der Hohlfaserkapillare 15 verbunden ist. Ist eine derartige Auslaßreduziereinrichtung nicht vorgesehen, was bei einigen Pumpen der Fall ist, wird der Anschluß der Hohlfaserkapillare dann natürlich an einem entsprechenden geeigneten Teil durchgeführt, was jedoch prinzipiell nichts an der zuvor beschriebenen Filterwirkung zur Vermeidung von Verstopfungen irgendwelcher Leitungsabschnitte innerhalb der Vorrichtung oder des Auslaßkatheters 8 ändert. Mithin wäre es grundsätzlich denkbar, daß auslaßseitig ein Ende der Hohlfaserkapillare 15 direkt an die Auslaßöffnung angeschlossen ist oder beispielsweise direkt an die ringförmige Kammer 9, falls auch irgendwelchen Gründen, wie gesagt, keine Auslaßreduziereinrichtung 7 vorgesehen sein sollte.

In jedem Falle ergibt sich der Vorteil, daß Verstopfungen durch Vorsehen des Hohfaserkapillarfilters 15 sicher vermieden werden, wobei dessen Anordnung funktionssicher und platzsparend vorgenommen werden kann.

## Patentansprüche

1. Implantierbare Vorrichtung (1) zur dosierten Abgabe von Medikamenten in den menschlichen Körper
- mit einer ersten Kammer (4) zur Speicherung des Medikamentes,
. die mit einer mittels eines durchstechbaren Septums (12) abgedichteten Nachfüllöffnung (12') versehen ist,
. die über eine Auslaßöffnung (6) und gegebenenfalls eine Auslaßreduziereinrichtung (7) mit einem Auslaßkatheter (8) verbunden ist, und
. die einen Filter zwischen Auslaßöffnung (6) und dem Innenraum der ersten Kammer (4) aufweist; und
- mit einer zweiten Kammer (5)
. die von der ersten Kammer (4) durch eine flexible Membran (3) getrennt ist, und
. die zum Aufbringen eines Druckes über die Membran (3) auf das in der ersten Kammer (4) befindliche Medikament dient, dadurch gekennzeichnet,
daß der Filter eine mikroporöse Hohlfaserkapillare (15) ist.

2. Implantierbare Vorrichtung (1) nach Anspruch 1, dadurch gekennzeichnet, daß im Falle des Vorsehens einer Auslaßreduziereinrichtung (7) das eine der Auslaßöffnung (6) benachbart angeordnete Ende dieser Auslaßreduziereinrichtung (7) mit einem der Enden der porösen Hohlfaserkapillare (15) verbunden ist.

3. Implantierbare Vorrichtung (1) nach Anspruch 1 oder 2, dadurch gekennzeichnet, daß die Hohlfaserkapillare (15) durch die Durchlaßöffnung (6) in die erste Kammer (4) geführt ist.

4. Implantierbare Vorrichtung (1) nach einem der Ansprüche 1 bis 3, dadurch gekennzeichnet, daß die Hohlfaserkapillare (15) im Inneren der ersten Kammer (4) in eine Nut (16) eingelegt ist, die oberhalb der Membran (3) angeordnet ist.

5. Implantierbare Vorrichtung (1) nach einem der Ansprüche 1 bis 4, dadurch gekennzeichnet, daß die Hohlfaserkapillare (15) in einer oder mehreren Windungen in der ersten Kammer (4) angeordnet ist.

6. Implantierbare Vorrichtung (1) nach einem der Ansprüche 1 bis 5, dadurch gekennzeichnet, daß die Hohlfaserkapillare (15) einen Innendurchmesser von etwa 200 »m und einen Außendurchmesser von etwa 280 »m aufweist.

7. Implantierbare Vorrichtung (1) nach einem der Ansprüche 1 bis 6, gekennzeichnet durch eine übliche Dialysatormembran aus Polysulfon (PS 600) oder eine zur Plasmafiltration oder Plasmafraktionierung verwendbare Membran höherer Ausschlußgrenze als Material für die Hohlfaserkapillare (15).

8. Implantierbare Vorrichtung (1) nach einem der Ansprüche 1 bis 7, dadurch gekennzeichnet, daß die Hohlfaserkapillare (15) einseitig geschlossen ist.

9. Implantierbare Vorrichtung (1) nach einem der Ansprüche 1 bis 8, dadurch gekennzeichnet, daß die Hohlfaserkapillare (15) mit dem Ende der Auslaßreduziereinrichtung (7) verklebt ist.

10. Implantierbare Vorrichtung (1) nach Anspruch 9, gekennzeichnet durch Polyurethan als Kleber.

## Claims

1. An implantable device (1) for the dosed administration of medicaments to the human body, comprising
- a first chamber (4) for storing a medicament
. said chamber being provided with a refill opening (12') sealed by means of a pierceable septum (12), and
. being connected via an outlet opening (6) and optionally via an outlet reducing means (7) to an outlet catheter (8), and
. being provided with a filter between said outlet opening (6) and the interior of said first chamber (4); and
- a second chamber (5)
. which is separated by a flexible diaphragm (3) from said first chamber (4) and
. which serves to apply pressure via said diaphragm (3) to said medicament provided in said first chamber (4),
characterized in that said filter is a microporous hollow-fiber capillary (15).

2. An implantable device (1) according to claim 1, characterized in that in case an outlet reducing means (7) is provided, the one end of said outlet reducing means (7) which is arranged adjacent said outlet opening (6) is connected to one of the ends of said porous hollow-fiber capillary (15).

3. An implantable device (1) according to claim 1 or 2, characterized in that said hollow-fiber capillary (15) is guided through the passage opening (6) into said first chamber (4).

4. An implantable device (1) according to any of claims 1 to 3, characterized in that said hollow fiber capillary (5) is inserted inside said first chamber (4) into a groove (16) which is arranged above said diaphragm (3).

5. An implantable device (1) according to any of claims 1 to 4, characterized in that said hollow-fiber capillary (15) is arranged in one or a plurality of windings in said first chamber (4).

6. An implantable device (1) according to any of claims 1 to 5, characterized in that said hollow-fiber capillary (15) has an inner diameter of about 200 »m and an outer diameter about 280 »m.

7. An implantable device (1) according to any of claims 1 to 6, characterized by a conventional dialyzer diaphragm of polysulfone (PS 600) or a diaphragm usable for plasma filtration or plasma fractionation, having a higher exclusion capacity, as material for the follow-fiber capillary (15).

8. An implantable device (1) according to any of claims 1 to 7, characterized in that said hollow-fiber capillary (15) is closed on one side.

9. An implantable device (1) according to any of claims 1 to 8, characterized in that said hollow-fiber capillary (15) is glued to the end of said outlet reducing means (7).

10. An implantable device (1) according to claim 9, characterized in that polyurethane is used as an adhesive.

## Revendications

1. Dispositif implantable (1) pour l'administration dosée de médicaments à l'homme, dispositif qui comprend les éléments suivants :
- une première chambre (4) comine réserve du médicament,
. avec une ouverture de remplissage (12') étanchéifiée par un septum perforable (12),
. reliée à un cathéter de décharge (8) par une ouverture de sortie (6) et le cas échéant un système de réduction de sortie (7), et
. qui comporte un filtre placé entre l'ouverture de sortie (6) et l'espace intérieur de la première chambre (4) ; et
- une seconde chambre (5),
. séparée de la première chambre (4) par une membrane souple (3), et
. servant à appliquer une pression, par l'intermédiaire de la membrane (3), sur le médicament qui se trouve dans la première chambre (4), dispositif caractérisé en ce que le filtre est un capillaire de fibre creuse microporeux (15).

2. Dispositif implantable (1) selon la revendication 1, caractérisé en ce que dans le cas où il comporte un système de réduction de sortie (7), une extrémité de celui-ci située près de l'orifice d'évacuation (6) est reliée à l'une des extrémités du capillaire de libre creuse microporeux (15).

3. Dispositif implantable (1) selon la revendication 1 ou 2, caractérisé en ce que le capillaire de libre creuse (15) va à la première chambre (4) en passant par l'orifice de passage (6).

4. Dispositif implantable (1) selon l'une des revendications 1 à 3, caractérisé en ce que le capillaire de fibre creuse (15) est placé à l'intérieur de la première chambre (4) dans une gorge (16) disposée au-dessus de la membrane (3).

5. Dispositif implantable (1) selon l'une des revendications 1 à 4, caractérisé en ce que le capillaire de fibre creuse (15) est disposé en un ou plusieurs enroulements dans la première chambre (4).

6. Dispositif implantable (1) selon l'une des revendications 1 à 5, caractérisé en ce que le capillaire de fibre creuse (15) a un diamètre intérieur d'environ 200 »m et un diamètre extérieur d'environ 280 »m.

7. Dispositif implantable (1) selon l'une des revendications 1 à 6, caractérisé en ce que le capillaire de fibre creuse (15) est une membrane de dialyseur ordinaire en polysulfone (PS 600) ou une membrane de filtration ou de fractionnement de plasma à plus haut seuil d'élimination.

8. Dispositif implantable (1) selon l'une des revendications 1 à 7, caractérisé en ce que le capillaire de fibre creuse (15) est fermé d'un côté.

9. Dispositif implantable (1) selon l'une des revendications 1 à 8, caractérisé en ce que le capillaire de fibre creuse (15) est collé à l'extrémité du système de réduction de sortie (7).

10. Dispositif implantable (1) selon la revendication 9, caractérisé en ce que l'adhésif est un polyuréthane.
